# EUROPÄISCHE PATENTSCHRIFT

(11) **EP 2 895 177 B1**
(45) Veröffentlichungstag und Bekanntmachung des Hinweises auf die Patenterteilung: **10.04.2019**
(21) Anmeldenummer: 13789478.8
(22) Anmeldetag: 11.09.2013
(51) Int. Cl.: A61K 35/02, A61P 1/00, A61P 17/00, A61P 39/04

(54) **VERWENDUNG EINES HUMINSÄUREPRÄPARATS ZUR BEHANDLUNG VON WARMBLÜTERN**
USE OF A HUMIC ACID PREPARATION FOR TREATING WARM-BLOODED ANIMALS
UTILISATION D'UNE PRÉPARATION D'ACIDE HUMIQUE POUR LE TRAITEMENT D'HOMÉOTHERMES

(30) Priorität: 11.09.2012 DE 102012108438
(43) Veröffentlichungstag der Anmeldung: 22.07.2015
(73) Patentinhaber: WH Pharmawerk Weinböhla GmbH, 01689 Weinböhla (DE)
(72) Erfinder: KÜHNERT, Manfred, 04299 Leipzig (DE); KRÜGER, Monika, 04103 Leipzig (DE); HAUFE, Svent, 01665 Diera-Zehren (DE); SHEATA, Awad, 04103 Leipzig (DE)
(74) Vertreter: Gottfried, Hans-Peter
(86) Internationale Anmeldenummer: PCT/DE2013/100324
(87) Internationale Veröffentlichungsnummer: WO 2014/040590

(56) Entgegenhaltungen:
- US-A1- 2009 204 187
- PIERLUIGI MAZZEI ET AL: "Quantitative Evaluation of Noncovalent Interactions between Glyphosate and Dissolved Humic Substances by NMR Spectroscopy", ENVIRONMENTAL SCIENCE & TECHNOLOGY, Bd. 46, Nr. 11, 5. Juni 2012 (2012-06-05), Seiten 5939-5946, XP055099825, ISSN: 0013-936X, DOI: 10.1021/es300265a
- ALBERS C N ET AL: "The influence of organic matter on sorption and fate of glyphosate in soil - Comparing different soils and humic substances", ENVIRONMENTAL POLLUTION, BARKING, GB, Bd. 157, Nr. 10, 1. Oktober 2009 (2009-10-01), Seiten 2865-2870, XP026423000, ISSN: 0269-7491 [gefunden am 2009-05-17]
- A. PICCOLO ET AL: "Adsorption of the herbicide glyphosate on a metal-humic acid complex", SCIENCE OF THE TOTAL ENVIRONMENT, Bd. 123-124, 1. August 1992 (1992-08-01), Seiten 77-82, XP055099787, ISSN: 0048-9697, DOI: 10.1016/0048-9697(92)90134-E
- MADHUN Y A ET AL: "BINDING OF IONIC AND NEUTRAL HERBICIDES BY SOIL HUMIC-ACID", SOIL SCIENCE SOCIETY OF AMERICA. JOURNAL, SOIL SCIENCE SOCIETY OF AMERICA, US, Bd. 50, Nr. 2, 1. Januar 1986 (1986-01-01) , Seiten 319-322, XP009175942, ISSN: 0361-5995
- W WU ET AL: "Effect of Natural Dissolved Humic Material on Bioavailability and Acute Toxicity of Fenpropathrin to the Grass Carp,Ctenopharyngodon idellus", ECOTOXICOLOGY AND ENVIRONMENTAL SAFETY, Bd. 42, Nr. 3, 6. März 1997 (1997-03-06), Seiten 203-206, XP055099875, ISSN: 0147-6513, DOI: 10.1006/eesa.1998.1743
- RITI SHARAN ET AL: "Inactivation and sub-lethal injury of Escherichia coli in a copper water storage vessel: effect of inorganic and organic constituents", ANTONIE VAN LEEUWENHOEK, KLUWER ACADEMIC PUBLISHERS, DO, Bd. 98, Nr. 1, 1. April 2010 (2010-04-01), Seiten 103-115, XP019786541, ISSN: 1572-9699
- DOBRANSKYTE A ET AL: "Effect of humic acid on water chemistry, bioavailability and toxicity of aluminium in the freshwater snail, Lymnaea stagnalis, at neutral pH", ENVIRONMENTAL POLLUTION, BARKING, GB, Bd. 140, Nr. 2, 1. März 2006 (2006-03-01), Seiten 340-347, XP028011988, ISSN: 0269-7491, DOI: 10.1016/J.ENVPOL.2005.06.030 [gefunden am 2006-03-01]
- N. T. L. TORSTENSSON ET AL: "Detoxification of glyphosate in soil", WEED RESEARCH, Bd. 17, Nr. 3, 1. Juni 1977 (1977-06-01), Seiten 209-212, XP055100018, ISSN: 0043-1737, DOI: 10.1111/j.1365-3180.1977.tb00468.x
- A. Piccolo ET AL: "Adsorption of Glyphosate by Humic Substances +", Journal of Agricultural and Food Chemistry, vol. 44, no. 8, 1 January 1996 (1996-01-01), pages 2442-2446, XP055323294, US ISSN: 0021-8561, DOI: 10.1021/jf950620x
- Monika Krüger ET AL: "Glyphosate suppresses the antagonistic effect of Enterococcus spp. on Clostridium botulinum", ANAEROBE, vol. 20, 6 February 2013 (2013-02-06), pages 74-78, XP055323223, GB ISSN: 1075-9964, DOI: 10.1016/j.anaerobe.2013.01.005

## Beschreibung

Die Erfindung betrifft die Verwendung eines Huminsäurepräparats Cellu-Ligno-Karbon-Isolat zur Anwendung bei der Behandlung von durch Glyphosat hervorgerufenen Erkrankungen bei Warmblütern, insbesondere wenn das Herbizid in Futterprodukten vorhanden ist.

Das Totalherbizid Glyphosat - allgemein unter dem Markennamen Roundup® bekannt - ist eine Entwicklung aus den 1970er Jahren. Glyphosat steht in der Anwendungshäufigkeit und in den Verkaufszahlen weltweit an erster Stelle. Seit 1996 sind importierte RR-(Roundup Ready)-Sojabohnen in Europa als Futter- und Lebendmittel ohne Kennzeichnungspflicht zugelassen. Erst 2004 erfolgte die Kennzeichnungspflicht als "GVO" (genveränderter Organismus). Der Wirkstoff ist universell einsetzbar. Er inaktiviert das Enzym 5-Enolpyruvyl- Shikimisäure-3-Phosphat-Synthase (EPSPS) und verhindert so die Synthese von aromatischen Aminosäuren (Phenylalanin, Tyrosin, Tryptophan) in Pflanzen und einigen Mikroorganismen, die dann absterben.

Ein Teil dieser Mikroorganismen gehört auch zur autochthonen Mikroflora und -fauna bzw. Mikrobiota des Magen-Darm-Traktes von Menschen und Tieren. Die Reduktion bzw. das gänzliche Fehlen der sogenannten gesundheitsfördernden Bakterien (z. B. Laktobazillen, Bifidobakterien, Bazillen, Enterokokken) oder ihrer Stoffwechselprodukte führen zu mikrobiellen Ungleichgewichten in diesem Organsystem, was schwerwiegende gesundheitliche Folgen nach sich ziehen kann, da einige Mikroorganismen wie Clostridium (C.) perfringens, C. tetani, C. botulinum, Salmonella Typhimurium tolerant bis resistent für Glyphosat sind und beim Wegfall der antagonistischen autochthonen Flora Erkrankungen mit diesen Erregern entstehen können.

EU-Grenzwerte für viele Futter- und Nahrungsmittel sind zwar festgelegt, doch geben sie keine Auskunft zur Wirkung von chronischen, oft lebenslänglichen Aufnahmen des Wirkstoffes auf die Organsysteme von Menschen und Tieren. Obwohl die von der EU postulierte tägliche verträgliche Glyphosat-Aufnahme bei 0,3 mg/kg Körpermasse liegt, fehlen Aussagen zu deren Folgen auf das Nerven-, Hormon-, Stoffwechsel- und Immunsystem bei Warmblütern.

Bisher werden folgende Glyphosat-Langzeitwirkungen von verschiedenen Arbeitsgruppen beschrieben: Genotoxizität, Kanzerogenität, Zytotoxizität und Teratogenität sowie Hormonabbauvorgänge. Weitere Effekte von Glyphosat richten sich bei Säugern auf Cytochrom p450, Aromatase, Östrogen-/ Testosteron-Rezeptoren sowie das Atmungs-, das hepatische und das Reproduktionssystem.

Die chemische Beschreibung des Herbizids und seiner Derivate ist bekannt. Es wird zur Behandlung von Nutzpflanzen zum Schutz vor Schädlingen und Unkraut verwendet. Dabei erfolgt eine breitflächige Aufbringung ohne besondere Schutzmaßnahmen und zugleich die ungewollte Beeinflussung der Qualität von rohen Ernteprodukten und damit von daraus hergestellten Futtermitteln und Lebensmitteln durch die Belastung mit dem Herbizid. Beschrieben werden Gesundheitsschäden bei Menschen und Tieren, die Reduzierung der Lebensmittel-, Futter- und Rohproduktqualitäten sowie Folgeschäden in der Nahrungskette.

Insbesondere im Körper von Warmblütern aufgenommene Konzentrationen werden über Ausscheidungsprodukte des Stoffwechsels ausgeschieden und belasten den Nährstoffkreislauf zusätzlich, wodurch es zu nachhaltigen Schäden bei Mikroorganismen im Boden und bei Säugern kommt. Die Aufnahme in den Nährstoffkreislauf führt zu einem zerstörerischen Eingriff in das Gleichgewicht der Pflanzengesundheit und zu einer Belastung des natürlichen Kreislaufs der Stoffe in der belebten Natur.

In der Literatur sind bisher keine Untersuchungsberichte bekannt, welche ein Verfahren oder ähnliches zur Inaktivierung der Stoffgruppe, zu der Glyphosat gehört, beschreiben. Ein Mittel oder ein Verfahren, das die vorgenannten Wirkungen insbesondere auf Warmblüter bzw. deren autochthoner Mikroflora und -fauna bzw. Mikrobiota hemmt oder unterbindet, ist gegenwärtig nach dem Stand der Technik nicht bekannt.

Bekannt sind nach dem Stand der Technik Huminsäuren. Die Druckschrift DE 196 50 317 C2 beschreibt ein Cellu-Ligno-Karbon-Isolat CLK-GA, auch als Leonardid bezeichnet, dessen Hauptfraktion natürliche Huminsäuren sind. Sie sind jener amorphe Anteil schwarzbraun bis braun gefärbter Stoffe in Böden, Ligniten, Torfen und Braunkohlen, welche im Zuge der sogenannten Humifizierung aus verrottendem organischem Material entstehen. Sie sind löslich in verdünnten Basen, in Mineralsäuren dagegen unlöslich. Natürliche (native) Huminsäuren können nicht als eindeutig definierte Substanzen im Sinne der klassischen Strukturchemie eingeordnet werden. Dies findet seinen Niederschlag darin, dass bis heute nur Näherungsmodelle für die chemische Struktur existieren. Zu den Bausteinen gehören neben Cellulose- und Ligninstrukturen auch polyionische Strukturen mit z. B. Karbonsäureester-, phenolischen Hydroxyl-, Karbonyl- und Karboxyl-Gruppen. Auch chinoide und flavonoide Strukturen sind vorhanden. Aus dem Stand der Technik sind weiterhin zahlreiche Betrachtungen zu Huminsäure, ihrem Vorkommen und ihren Effekten bekannt.

Die Druckschrift US 2009/204187 A1 befasst sich allgemein mit der Verwendung einer Art Heilerde, die neben anderen Komponenten auch allgemein Huminsäuren enthält, zur Eliminierung von Toxinen aus dem Körper eines Säugetiers. Soweit isolierte Huminsäuren Bestandteil der Zubereitung sind, erfolgt die Isolierung aus einem Boden (vgl. Abs. 0015 ff.), allenfalls noch aus Torf (vgl. Abs. 0023), nicht jedoch als Cellu-Ligno-Karbon-Isolat.

Weiterhin kann das in der Druckschrift US 2009/204187 A1 dargestellte breite Spektrum an Toxinen, die eliminiert werden sollen, gerade Glyphosat nicht umfassen, da dieses zum Zeitpunkt der Veröffentlichung als Toxin im Zusammenhang mit tierischen Lebewesen ausdrücklich nicht in Erscheinung trat. Vielmehr gilt es als schnell und rückstandsfrei abbaubar, so dass etwaige Nebenwirkungen in Nahrungs- oder Futtermitteln nicht in Betracht gezogen wurden und für etwaige Maßnahmen hiergegen keine Veranlassung bestand.

In den Veröffentlichungen PIERLUIGI MAZZEI ET AL 2012 (PIERLUIGI MAZZEI ET AL: "Quantitative Evaluation of Noncovalent Interactions between Glyphosate and Dissolved Humic Substances by NMR Spectroscopy", ENVIRONMENTAL SCIENCE & TECHNOLOGY, Bd. 46, Nr. 11, 5. Juni 2012 (2012-06-05), S. 5939-5946, XP055099825, ISSN: 0013-936X, DOI: 10.1021/es300265a) und MADHUN Y A ET AL 1986 (MADHUN Y A ET AL: "BINDING OF IONIC AND NEUTRAL HERBICIDES BY SOIL HUMIC-ACID", SOIL SCIENCE SOCIETY OF AMERICA. JOURNAL, SOIL SCIENCE SOCIETY OF AMERICA, US, Bd. 50, Nr. 2, 1. Januar 1986 (1986-01-01), Seiten 319-322, XP009175942, ISSN: 0361-5995) wird über Untersuchungen zur Interaktion zwischen Glyphosat und löslichen Fulvosäuren und Huminsäuren bzw. einer Bindungswirkung berichtet. Im Ergebnis wurde festgestellt, dass sich Glyphosat spontan und signifikant an lösliche Huminstoffe zu binden vermag (vgl. Abstract PIERLUIGI MAZZEI ET AL 2012). Hieraus resultiert die Erkenntnis, dass sich eine Wassergefährdung durch diese Huminstoffe ergibt, wenn sie durch das Bodenprofil in ein Gewässer wandern.

Zugleich wird aber in Einklang mit der allgemein üblichen Beurteilung festgestellt, dass es sich bei Glyphosat um ein vergleichsweise wenig giftiges Herbizid handelte und nur der ständige Eintrag in die Gewässer problematisch ist (vgl. PIERLUIGI MAZZEI ET AL 2012 S. 5939, Spalte 1 unten). Die Probleme durch Rückstände in geringen Dosen, die weit davon entfernt sind, eine unmittelbar toxische Wirkung zu entfalten, in ihren nachteiligen mittelbaren Wirkungen über den Einfluss auf die Mikrobiologie zu betrachten, ist hiervon nicht berührt.

Die in der Veröffentlichung ALBERS C N ET AL 2009 (ALBERS C N ET AL: "The influence of organic matter on sorption and fate of glyphosate in soil - Comparing different soils and humic substances", ENVIRONMENTAL POLLUTION, BARKING, GB, Bd. 157, Nr. 10, 1. Oktober 2009 (2009-10-01), Seiten 2865-2870, XP026423000, ISSN: 0269-7491 [gefunden am 2009-05-17]) beschriebenen Untersuchungen richten sich auf die Aufnahme von Glyphosat in Böden und humiden Substanzen, die auch Huminsäuren enthalten. Auch isolierte Huminsäuren wurden untersucht und dabei festgestellt, dass ein Bindungsmechanismus existiert. Da keine Hinweise auf die Wirkungen der isolierten Huminsäuren allgemein, geschweige denn Cellu-Ligno-Karbon-Isolat, in der Offenbarung enthalten sind, gilt das oben zu PIERLUIGI MAZZEI ET AL 2012 Ausgeführte entsprechend.

Die Veröffentlichung A. PICCOLO ET AL 1992 (A. PICCOLO ET AL: "Adsorption of the herbicide glyphosate on a metal-humic acid complex", SCIENCE OF THE TOTAL ENVIRONMENT, Bd. 123-124, 1. August 1992 (1992-08-01), Seiten 77-82, XP055099787, ISSN: 0048-9697, DOI: 10.1016/0048-9697(92)90134-E) befasst sich speziell mit Metall-Huminsäure-Verbindungen, insbesondere Eisen, in Zusammenhang mit der Adsorption von Glyphosat. Insbesondere die Verwendung von speziellen Huminsäuren wie Cellu-Ligno-Karbon-Isolat wird jedoch nicht betrachtet.

W WU ET AL 1997 (W WU ET AL: "Effect of Natural Dissolved Humic Material on Bioavailability and Acute Toxicity of Fenpropathrin to the Grass Carp, Ctenopharyngodon idellus", ECOTOXICOLOGY ANO ENVIRONMENTAL SAFETY, Bd. 42, Nr. 3, 6. März 1997 (1997-03-06), S. 203-206, XP055099875, ISSN: 0147-6513, DOI: 10.1006/eesa.1998.1743) und DOBRANSKYTE A ET AL 2006 (DOBRANSKYTE A ET AL: "Effect of humic acid on water chemistry, bioavailability and toxicity of aluminium in the freshwater snail, Lymnaea stagnalis, at neutral pH", ENVIRONMENTAL POLLUTION, BARKING, GB, Bd. 140, Nr. 2, 1. März 2006 (2006-03-01), Seiten 340-347, XP028011988, ISSN: 0269-7491, DOI: 10.1016/J.ENVPOL.2005.06.030 [gefunden am 2006-03-01]) befassen sich in Zusammenhang mit der Wirkung von Huminsäure nicht mit einer Verringerung einer antimikrobiellen Aktivität. Denn im Gegensatz zu Fenpropathrin gilt Glyphosat nicht als antibakteriell. Untersucht werden zudem wässrige Huminsäuren, allerdings unspezifiziert und im Sinne einer Simulation natürlicher Gewässer, die einen Gehalt an gelösten Huminstoffen aufweisen. Dabei wurde gezeigt, dass in dem Wasser lebende Fische umso weniger Fenpropathrin aufnehmen, je höher der Gehalt an gelösten Huminstoffen ist und einen Teil des Fenpropathrins bindet.

Durch RITI SHARAN ET AL 2010 (RITI SHARAN ET AL: "Inactivation and sub-lethal injury of Escherichia coli in a copper water storage vessel: effect of inorganic and organic constituents", ANTONIE VAN LEEUWENHOEK, KLUWER ACADEMIC PUBLISHERS, DO, Bd. 98, Nr. 1, 1. April 2010 (2010-04-01), Seiten 103-115, XP019786541, ISSN: 1572-9699) werden Kupferderviate und deren antimikrobieller Aktivität näher betrachtet. Jedoch gilt im Gegensatz zu Cu-Derivaten Glyphosat nicht als antibakteriell. Im Übrigen richten sich die Untersuchungen auf ein sehr breites Spektrum organischer Stoffe (vgl. S. 1, rechte Spalte, Zeilen 6 und 7), die durch ganz unterschiedliche Effekte auf das untersuchte Bakterium E. coli wirken können. Eine komplexe Wirkung kann zudem durch die Betrachtung eines einzelnen Bakteriums nicht nahegelegt werden kann.

Die in der Veröffentlichung N T L TORSTENSEN ET AL 1977 (N. T. L. TORSTENSSON ET AL: "Detoxification of glyphosate in soil", WEED RESEARCH, Bd. 17, Nr. 3, 1. Juni 1977 (1977-06-01), Seiten 209-212, XP0551 00018, ISSN: 0043-1737, DOI: 10.1111/j. 1365-3180.1977.tb00468.x) dargestellte Auswertung der Untersuchungen in der Zusammenfassung legt nahe, dass der Abbau von Glyphosat im Boden im Wesentlichen auf einen bakteriellen Abbau zurückgeführt wird und die Adsorption für eine schnelle anfängliche Inaktivierung verantwortlich ist.

Besonders die aus pflanzlichen Produkten entstandenen Huminsäuren, zu denen auch die Braunkohle-Huminsäuren gehören, weisen außer ihrem aromatischphenolischen Grundaufbau Flavonstrukturen auf (Fisetin, Quercetin, Flavone, Xanthine).

Als Ausgangsstoff zur Gewinnung der erfindungsgemäßen CLK-Isolate werden vorzugsweise Alkali-Huminat-Huminsäure-Gemische eingesetzt, wie sie beispielsweise gemäß den Angaben in der Druckschrift DD 44 155 aus die Huminstoffe in ausreichender Menge enthaltenden Naturstoffen erhältlich sind. Weiterhin sollten die huminstoffhaltigen Naturstoffe einen möglichst geringen, vorzugsweise gegen Null gehenden Gehalt an für Mensch und Tier schädlichen Bestandteilen aufweisen. Solche Bestandteile können beispielsweise akut oder chronisch toxisch sowie genotoxisch, teratogen, mutagen oder kanzerogen sein. Huminsäuren des zuvor beschriebenen Typs sind physikalisch-chemisch aufgearbeitete natürliche Huminsäuren, die aus einer speziell ausgewählten Braunkohlenlagerstätte gewonnen werden, vorzugsweise Braunkohle aus Lagen, die nur einen geringen oder gar keinen Gehalt an den oben genannten, schädlichen Bestandteilen aufweist. Darüber hinaus können auch Torfe, Moor- und Seesedimente eingesetzt werden.

Eine weitere Veröffentlichung A. Piccolo ET AL: "Adsorption of Glyphostae by Humic Substances +", Journal of Agricultural and Food Chemistry, Bd. 44, Nr. 8, 1. Januar 1996 (1996-01-01), S. 2442-2446, XP055323294, US, ISSN: 0021-8561, DOI: 10.1021/jf950620x befasst sich mit aus Lignite isolierten Huminsäuren, die Glyphosat binden können. Welche Effekte hierdurch im Weiteren erzielt werden können, wird jedoch nicht beschrieben.

Einen weiteren Beitrag zum der vorliegenden Erfindung zugrundeliegenden Stand der Technik leistet die Veröffentlichung Monika Krüger ET AL: "Glyphosate suppresses the antagonistic effect of Enterococcus spp. on Clostridium botulinum", ANAEROBE, Bd. 20, 6. Februar 2013 (2013-02-06), Seiten 74-78, XP055323223, GB, ISSN: 1075-9964, DOI: 10.1016/j.anaerobe.2013.01.005*.* Darin werden mikrobielle Effekte aufgeklärt, die zu Erkrankungen insbesondere des Magen-Darm-Kanals nach der Aufnahme von Glyphosat führen.

Die Aufgabe der vorliegenden Erfindung ist es, die Wirkung von im Futter befindlichem Glyphosat und seiner Derivate im Körper, in Stoffwechselausscheidungsprodukten und im Boden, vor allem aber auf die Magen-Darm-Mikrobiose sowie auf weitere Körpersysteme, zu reduzieren oder zu neutralisieren, um die mikrobiologische Aktivität im Körper, im Boden und bei der Behandlung von Stoffwechselausscheidungsprodukten jeder Art zu erhalten.

Die Aufgabe wird gelöst durch die Anwendung eines Huminsäurepräparats Cellu-Ligno-Karbon-Isolat zur Behandlung von Erkrankungen bei Warmblütern, die durch das Pflanzenschutzmittel Glyphosat hervorgerufen wurden, wobei das Pflanzenschutzmittel Glyphosat an das Huminsäurepräparat gebunden wird und die wachstumshemmende Wirkung des Pflanzenschutzmittels Glyphosat auf physiologische Keime im Magen-Darm-Trakt gemindert wird, wobei das Pflanzenschutzmittel Glyphosat in Nahrungs- und Futterprodukten vorhanden ist. Das Huminsäurepräparat ist in der Weise zur oralen Aufnahme vorgesehen, dass es zur Verbesserung oder zum Erhalt des Gesundheitszustandes des Magen-Darm-Kanals führt.

Zum Nachweis der Glyphosat neutralisierenden Wirkung von CLK-GA wurden die Indikatorkeime *Enterococcus faecalis* und *Bacillus badius* eingesetzt. Der Erreger *Enterococcus faecalis* wurde aus der Mikroalge Chlorella vulgaris auf Citrat-Azid-Tween-Carbonat (CATC)-Agar isoliert. Verdächtige Kolonien (Gram-positive, Katalasenegative Kokken) wurden auf Blutagarplatten (Oxoid) überimpft und mittels biochemischer Tests differenziert. Die Speziesdifferenzierung wurde mittels MALDI-TOF-MS bestätigt. Die Isolierung des Erregers *Bacillus badius* erfolgte aus *Chlorella vulgaris* auf Blutagar (Oxoid).

Die Speziesdifferenzierung wurde anhand biochemischer Parameter durchgeführt. Die Bestätigung erfolgte mittels MALDI-TOF-MS. Zur Untersuchung wurde das kommerzielle Glyphosat-Präparat Roundup UltraMax® (450g/l Glyphosat als Isopropylamin-Salz in SL-Formulierung) verwendet. Der Wert für die minimale Hemmstoffkonzentration von Glyphosat für die Indikatorkeime (MHK) wurde mittels Mikrodilutionsverfahren (MIO) gemäß den aktuellen Vorgaben des National Committee for Clinical Laboratory Standards (NCCLS) bestimmt. Sie wurde bei *E. faecalis* und *B*. *badius* in RCM-Bouillon (SIFIN) durchgeführt. Die Bestimmung des neutralisierenden Effekts des Huminsäureproduktes CLK-GA für Glyphosat erfolgte über den Glyphosat-MHK. Die MHK-Bestimmungen für Glyphosat und Glyphosat-CLK-GA-Gemische erfolgte in RCM-Bouillon, wie oben beschrieben. CLK-GA wurde in den Konzentrationen 1,0; 0,5 und 0,25 mg/ml verwendet. Die Neutralisation von Glyphosat wurde in den Konzentrationen von 300 µg/ml, 600 µg/ml, 1200 µg/ml und 2400 µg/ml mit 1 mg/ml, 0,5 mg/ml und 0,25 mg/ml CLK-GA in RCM-Bouillon gemischt und über Nacht bei 37 °C durchgeführt. Nach Zentrifugation (3000 U/min) und Filtration der einzelnen Ansätze durch einen Zentrifugal-Filter (3 Kda, VWR, Mexico) wurden zu diesen 104/ml KbE *E. faecalis* bzw. *B*. *badius* dazu gegeben. Nach Wachstum über Nacht bei 37 °C wurde der Inhalt jedes Röhrchens auf CATC-Agar oder Blutagar ausgestrichen und nach 48h bewertet.

Die Untersuchungen erbrachten folgende Ergebnisse. Der MHK von *E*. *faecalis* liegt bei 200 µg/ml, der MHK-Wert für *B*. *badius* liegt bei 150 µg/ml Glyphosat. Die MHK-Werte von Glyphosat und den Glyphosat-CLK-GA-Gemischen für *E. faecalis* und *B*. *badius* sind in Tabelle 1 dargestellt. Diese Werte wurden durch das Huminsäureprodukt CLK-GA in der Konzentration von 1 mg/ml auf 2400 µg/ml für beide Bakterienspezies erhöht. Mit 0,5 mg/ml und 0,25 mg/ml CLK-GA waren noch Glyphosatkonzentrationen von 600 µg/ml neutralisierbar. Der Zusatz eines Glyphosat-CLK-GA-Gemisches mit einer Endkonzentration von 300 µg/ml Glyphosat und 1 mg/ml CLK-GA zu einer 10⁴ KbE/ml Enterokokken- bzw. Bacillussuspension führte bei beiden Erregersuspensionen nicht zur Eliminierung der Keime. In den Glyphosat-Kontrollen blieb das Wachstum aus, die CLK-GA-Wachstumskontrollen ohne Glyphosat enthielten ähnliche Keimzahlen wie die Glyphosat-CLK-GA-Gemische.

Die erfindungsgemäßen Wirkungen und Vorteile zeigen sich bei der Anwendung von Huminsäurepräparaten allgemein, besonders deutlich jedoch, wenn als Huminsäurepräparat ein Cellu-Ligno-Karbon-Isolat (CLK-GA), gewonnen gemäß vorstehender Beschreibung (vgl. auch Druckschrift DE 196 50 317 C2), zum Einsatz kommt.

**Tabelle 1 zeigt die MHK-Werte von Glyphosat und Glyphosat-CLK-GA-Gemisch für Enterococcus feacalis und Bacillus badius.**

| Indikatorkeime | MHK-Werte | |
|---|---|---|
| | Glyphosat | Glyphosat-CLK-GA-Gemisch |
| *E.fecalis* | 200 µg/ml | 2400 µg/ml |
| *Bacillus badius* | 150 µg/ml | 2400 µg/ml |

Die MHK-Werte für *E. faecalis* steigen in Glyphosat-CLK-GA-Gemischen von 200 µg/ml auf 2400 µg/ml (12-fach) und für *B*. *badius* von 150 µg/ml auf 2400 µg/ml (16-fach). Die Bindung von Glyphosat an das Huminsäureprodukt CLK-GA ist konzentrationsabhängig.

Die Wirkungen sind besonders deutlich, wenn das Pflanzenschutzmittel ein Totalherbizid aus der Gruppe der Phosphanate und seiner Derivate, insbesondere Glyphosat, ist. Dies wurde mit den oben beschriebenen Untersuchungen gezeigt. Glyphosat findet besonders breiten Einsatz

Besonders vorteilhaft ist es, wenn als Huminsäurepräparat ein Cellu-Ligno-Karbon-Isolat zum Einsatz kommt, dessen Herstellung bereits etabliert und das somit kostengünstig verfügbar ist.

Es hat sich als besonders wirkungsvoll erwiesen, wenn das Huminsäurepräparat in der Weise zur oralen Aufnahme vorgesehen ist, dass sie zur Verbesserung oder zum Erhalt des Gesundheitszustandes des Magen-Darm-Kanals (Schleimhaut) führt. Diese Wirkung tritt beispielsweise ein bei Wiederkäuern, Schweinen, Geflügel und anderen Warmblütern. Bereits nach mehrwöchiger Zugabe des Huminsäurepräparats zum Futter bessern sich ein insgesamt schlechter Allgemeinzustand der Eltern- und Jungtiere von Rindern sowie eine schlechte Milchqualität so weit, dass ein normaler Gesundheitszustand und einer normale Milchqualität zu verzeichnen sind. Insbesondere wird durch die Behandlung auch die Qualität von Lebensmitteln, die von belasteten Säugern stammen, hier beispielsweise die Milch, verbessert.

Entsprechendes gilt für die Verwendung bei Erkrankungen des Menschen, die auf den Verzehr belasteter Nahrungsmittel zurückzuführen sind. Eine Anwendung ist jedoch auch für in Prophylaxe, Metaphylaxe, Therapie und Regeneration in Human- und Veterinärmedizin vorgesehen. Hierdurch wird der Erhalt der mikrobiologischen Aktivität im Körper, die durch die Wirkungen des Pflanzenschutzmittels Glyphosat beeinträchtigt wird, gesichert.

Als besonders vorteilhaft hat es sich erwiesen, wenn das Huminsäurepräparat bereits bei der Herstellung von Nahrungs- oder Futtermitteln zur Verbesserung der Rohprodukte eingesetzt und das Pflanzenschutzmittel an das Huminsäurepräparat gebunden wird. Damit können mit Pflanzenschutzmittel belastete Partien dennoch der Verwertung als Futter bzw. Nahrungsmittel zugeführt werden, ohne nachteilige Folgen befürchten zu müssen. Die Qualität der Lebensmittel, Futtermittel bzw. Rohprodukte wird gesteigert. Besonders günstig ist ein Einsatz bereits an dieser Stelle der Nahrungskette deshalb, weil Folgeschäden in der Nahrungskette vermieden werden.

Eine weitere vorteilhafte Verwendung besteht darin, dass das Huminsäurepräparat den Stoffwechselausscheidungsprodukten oder deren Folgeprodukten bzw. Verarbeitungsstufen zugeführt und das Pflanzenschutzmittel an das Huminsäurepräparat gebunden wird. Da die Ausscheidungsprodukte des Stoffwechsels von Nutztieren in der Regel als Dünger wieder auf den Boden aufgebracht werden, wird dieser mit den über die Nahrung in den Kreislauf gelangten Pflanzenschutzmitteln zusätzlich belastet. Diese Belastung mit Pflanzenschutzmitteln beeinträchtigt das Bodenleben, denn die für die Prozesse im Boden, beispielsweise die Humusbildung als wichtigem Faktor der Bodenfruchtbarkeit, verantwortlichen Mikroorganismen werden gestört. Wird das unerwünschte Pflanzenschutzmittel jedoch in seiner Wirkung "neutralisiert", indem es durch die Huminsäure in den Ausscheidungsprodukten gebunden wird, bleibt diese nachteilige Wirkung aus mit der Folge einer gesunden, natürlichen Entwicklung des Bodenlebens.

Die Wirkung der Ausscheidungsprodukte, beispielsweise Rohgülle, auf das Bodenleben nach der Ausbringung entspricht der von Folgeprodukten, wie beispielsweise in einer Biogasanlage behandelter Gülle (Biogasgülle), in Bezug auf den Gehalt an in den Kreislauf gelangten Pflanzenschutzmitteln. Somit ist im Interesse eines hochwertigen Düngerprodukts auch Biogasgülle einer Behandlung durch das Huminsäurepräparat zu unterziehen.

Vorteilhafterweise erfolgt die Behandlung jedoch, bevor die Ausscheidungsprodukte in die Biogasanlage gelangen. Denn auch dort kann die empfindliche Population von Mikroorganismen durch den Einfluss des Pflanzenschutzmittels gestört werden und dadurch die Funktion, zumindest aber Produktivität und Stabilität des Biogasprozesses beeinträchtigt werden. Dies wird durch eine erfindungsgemäße Behandlung der Ausscheidungsprodukte vorab mit dem Huminsäurepräparat vermieden. Alternativ wird das Huminsäurepräparat unmittelbar auf belasteten Boden ausgebracht und das Pflanzenschutzmittel an das Huminsäurepräparat gebunden wird. Die Wirkung auf das Bodenleben entspricht der vorgenannten, wobei auch bei bereits geschädigtem Bodenleben eine Verbesserung zu erwarten ist. Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
Fig. 1: Konzentrationsabhängige Neutralisation von Glyphosat durch das Huminsäureprodukt CLK-GA im Indikatorsystem *E. faecalis;*
Fig. 2: Konzentrationsabhängige Neutralisation von Glyphosat durch das Huminsäureprodukt CLK-GA im Indikatorsystem *B. badius;* und
Fig. 3: Ergebnis der Kultivierung von Enterokokkensuspensionen (104/ml) nach Kultivierung mit Filtraten eines 600 µg/ml Glyphosat -1mg/ml CLK-GA-Gemisches in der Endkonzentration 300 µg Glyphosat/ ml.

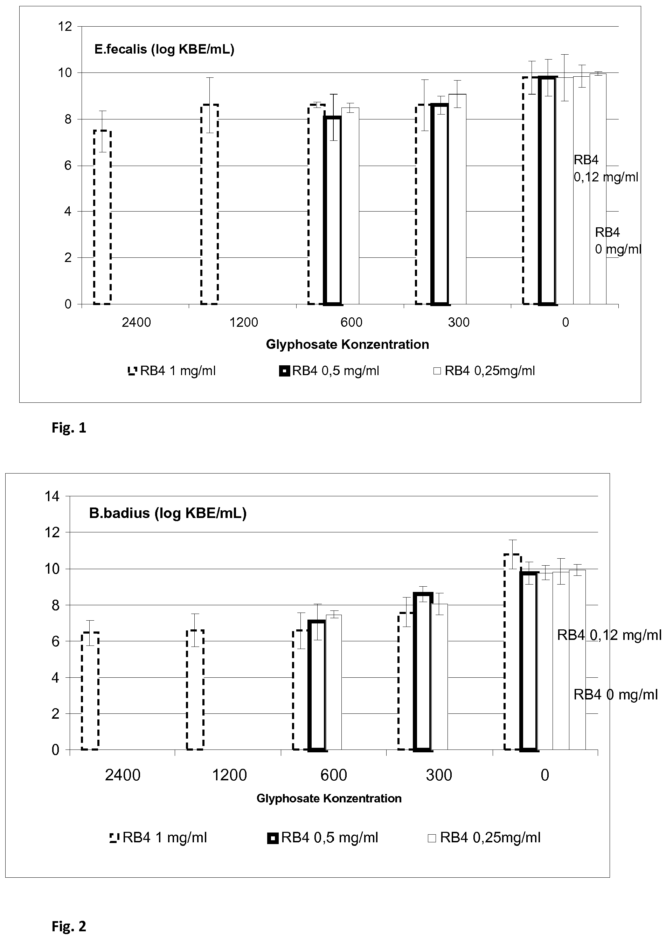

Fig. 1 zeigt die konzentrationsabhängige Neutralisation von Glyphosat durch das Huminsäureprodukt CLK-GA (in den Figuren entsprechend dem Material aus der dokumentierten Versuchsreihe als "RB4" bezeichnet) im Indikatorsystem *E. faecalis* und Fig. 2 zeigt die konzentrationsabhängige Neutralisation von Glyphosat durch das Huminsäureprodukt CLK-GA im Indikatorsystem *B. badius.* Die Angabe CLK-GA 0,12 mg/ ml in der Gruppe "Glyphosate Konzentration 0" von Fig. 1 bezieht sich auf den vorletzten Balken, die Angabe CLK-GA 0 mg/ml auf den letzten Balken. Bei Fig. 2 bezieht sich entsprechend die Angabe CLK-GA 0,12 mg/ml auf den vorletzten Balken, die Angabe CLK-GA 0 mg/ml auf den letzten Balken der Gruppe "Glyphosate Konzentration 0".

Der Wert für die minimale Hemmstoffkonzentration (MHK) von *E. faecalis* liegt bei 200µg/ml, der MHK-Wert für *B*. *badius* liegt bei 150µg/ml Glyphosat. Diese Werte wurde durch das Huminsäureprodukt CLK-GA in der Konzentration von 1 mg/ml auf 2400µg/ml für beide Bakterienspezies erhöht. Mit 0,5 mg/ml und 0,25 mg/ml CLK-GA waren noch Glyphosatkonzentrationen von 600 µg/ml neutralisierbar.

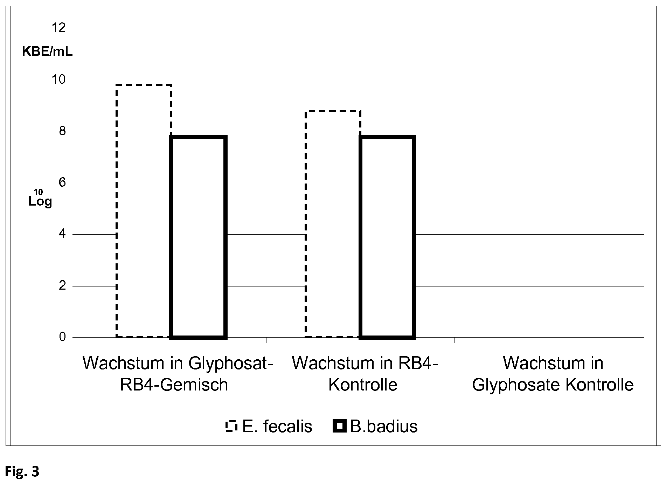

Fig. 3 zeigt das Ergebnis der Kultivierung von Enterokokkensuspensionen (104/ml) nach Kultivierung mit Filtraten eines 600 µg/ml Glyphosat -1 mg/ml CLK-GA-Gemisches in der Endkonzentration 300 µg Glyphosat/ml. Der Zusatz eines Glyphosat-CLK-GA-Gemisches mit einer Endkonzentration von 300 µg/ml Glyphosat und 1 mg/ml CLK-GA WH67® zu einer 104 KbE/ml Enterokokken- bzw. Bacillussuspension führte bei beiden Erregersuspensionen nicht zur Eliminierung der Keime.

In den Glyphosat-Kontrollen blieb das Wachstum aus, die CLK-GA-Wachstumskontrollen (ohne Glyphosat) enthielten ähnliche Keimzahlen wie die Glyphosat-CLK-GA-Gemische.

Laborversuche zeigen, dass die Neutralisierungswirkung von CLK-GA für die antimikrobielle Wirkung des Glyphosat auf *E. faecalis* in Glyphosat-CLK-GA WH67®-Gemischen (1 mg/ml CLK-GA) und verschiedene Konzentrationen von Glyphosat nachweisbar sind.

Bei einer Anwendung in vivo mit Einsatz von CLK-GA in einem stark gesundheitsgeschädigten Milchviehbetrieb durch Glyphosat belastetes Futter zeigte sich folgender Ausgangszustand:
- schlechter Allgemeinzustand der Eltern- und Jungtiere,
- trotz regelmäßiger Klauenpflege schlechter Zustand,
- schlechte Fruchtbarkeit,
- Qualität der Milch sehr eingeschränkt (Salmonellenzahl >70),
- äußerer Zustand der Tiere schmutzig, struppig,
- Jungtiere anfällig für Durchfälle,
- Fressunlust und Durchfälle,
- Todesfälle und
- neurologische, nervaleAusfallerscheinungen.

Die Behandlung erfolgte durch Einmischung von CLK-GA in das Futter über 4-6 Wochen.

Hierauf zeigten sich die folgenden klinischen Ergebnisse:
- die Tiere fraßen wieder normal,
- der äußere Zustand wurde besser (Fell glatt und glänzend),
- Klauengesundheit verbesserte sich und Pflegeintervalle vergrößerten sich,
- Jung- und Alttiere hatten keine Durchfälle mehr,
- keine Todesfälle,
- normalisiertes Sozialverhalten,
- keine neurologischen, nervalen Ausfallerscheinungen zu beobachten und
- Salmonellenzahl <5.

Bei Laboruntersuchungen zeigten sich zudem normalisierte Leberwerte.

### Bezugszeichenliste

- 1: Wachstum in Glyphosat CLK-GA-Gemisch
- 2: Wachstum in CLK-GA-Kontrolle
- 3: Wachstum in Glyphosat-Kontrolle

## Patentansprüche

1. Huminsäurepräparat Cellu-Ligno-Karbon-Isolat zur Anwendung bei der Behandlung von durch ein Pflanzenschutzmittel Glyphosat hervorgerufenen Erkrankungen bei Warmblütern, **dadurch gekennzeichnet, dass** das Pflanzenschutzmittel Glyphosat an das Huminsäurepräparat gebunden und die wachstumshemmende Wirkung des Pflanzenschutzmittels Glyphosat auf physiologische Keime im Magen-Darm-Trakt gemindert wird, wobei das Pflanzenschutzmittel Glyphosat in Nahrungs- und Futterprodukten vorhanden ist und das Huminsäurepräparat in der Weise zur oralen Aufnahme vorgesehen ist, dass es zur Verbesserung oder zum Erhalt des Gesundheitszustandes des Magen-Darm-Kanals führt.

2. Huminsäurepräparat zur Anwendung nach Anspruch 1, wobei das Pflanzenschutzmittel Glyphosat in Nahrungs- und Futterrohprodukten vorhanden ist und das Huminsäurepräparat bereits bei der Herstellung von Nahrungs- oder Futtermitteln zur Verbesserung der Qualität der Rohprodukte eingesetzt und das enthaltene Pflanzenschutzmittel Glyphosat an das Huminsäurepräparat gebunden wird.

3. Huminsäurepräparat zur Anwendung nach Anspruch 1, wobei das Huminsäurepräparat den Stoffwechselausscheidungsprodukten oder deren Folgeprodukten zugeführt und das Pflanzenschutzmittel Glyphosat an das Huminsäurepräparat gebunden wird.

4. Huminsäurepräparat zur Anwendung nach Anspruch 1, wobei das Huminsäurepräparat auf belasteten Boden ausgebracht und das Pflanzenschutzmittel Glyphosat an das Huminsäurepräparat gebunden wird.

## Claims

1. A humic acid preparation lignocellulose carbon isolate for use in the treatment of diseases in warm-blooded animals caused by a plant protection agent glyphosate, **characterized in that** the plant protection agent glyphosate is bonded to the humic acid preparation and the growth-inhibiting effect of the plant protection agent glyphosate on physiological microbes in the gastrointestinal tract is reduced, wherein the plant protection agent glyphosate is present in food and animal feed products and the humic acid preparation is for oral intake such that it results in improvement or maintenance of the state of health of the gastrointestinal tract.

2. The humic acid preparation for use according to claim 1, wherein the plant protection agent glyphosate is present in food and animal feed raw products, and the humic acid preparation is already employed to improve the quality of the raw products in the production of food or animal feed products and the contained plant protection agent glyphosate is bonded to the humic acid preparation.

3. The humic acid preparation for use according to claim 1, wherein the humic acid preparation is added to the metabolic excretory products or their secondary products and the plant protection agent glyphosate is bonded to the humic acid preparation.

4. The humic acid preparation for use according to claim 1, wherein the humic acid preparation is applied to contaminated soil and the plant protection agent glyphosate is bonded to the humic acid preparation.

## Revendications

1. Préparation d'acide humique isolat de cellu-ligno-carbone pour l'application lors du traitement de maladies provoquées par un agent protecteur de plantes glyphosate chez des animaux à sang chaud, **caractérisée en ce que** l'agent protecteur de plantes glyphosate est fixé à la préparation d'acide humique et l'action inhibitrice de croissance de l'agent protecteur de plantes glyphosate est réduite sur des germes physiologiques dans le tractus gastro-intestinal, dans laquelle l'agent protecteur de plantes glyphosate est présent dans des produits alimentaires et fourragers et la préparation d'acide humique est prévue pour l'absorption orale de telle sorte qu'elle conduit à l'amélioration ou à la préservation de l'état de santé du canal gastro-intestinal.

2. Préparation d'acide humique pour l'application selon la revendication 1, dans laquelle l'agent protecteur de plantes glyphosate est présent dans des produits alimentaires et fourragers et la préparation d'acide humique est déjà utilisée lors de la fabrication d'aliments ou de matières fourragères pour l'amélioration de la qualité des produits bruts et l'agent protecteur de plantes glyphosate contenu est fixé à la préparation d'acide humique.

3. Préparation d'acide humique pour l'application selon la revendication 1, dans laquelle la préparation d'acide humique est amenée aux produits d'élimination métabolique ou leurs produits dérivés et l'agent protecteur de plantes glyphosate est fixé à la préparation d'acide humique.

4. Préparation d'acide humique pour l'application selon la revendication 1, dans laquelle la préparation d'acide humique est épandue sur un sol chargé et l'agent protecteur de plantes glyphosate est fixé à la préparation d'acide humique.
